# EUROPEAN PATENT APPLICATION

(11) **EP 1 920 771 A2**
(43) Date of publication of application: **14.05.2008**
(21) Application number: 07022682.4
(22) Date of filing: 26.04.2002
(51) Int. Cl.: A61K 31/38, A61K 47/44, A61P 3/06

(54) **Methods of treating hyperlipidemia**

(30) Priority: 03.05.2001 US 848159
(62) Divisional of application: 02729013.9
(71) Applicant: ALLERGAN, INC., Irvine, California 92612 (US)
(72) Inventor: Yuan, Yang-Dar, Irvine CA 92612 (US); Thacher, Scott M., Costa Mesa CA 92627 (US); Klein, Elliot S., Danbury CT 06180 (US); Chandraratna, Roshantha A., Laguna Hills CA 92653 (US)
(74) Representative: HOFFMANN EITLE

(57) **Abstract**

The current invention relates to the use of an RAR antagonist or an RAR inverse agonist for the manufacture of a medicament for the treatment of hyperlipidemia in a mammal and to an RAR antagonist or an RAR inverse agonist for use in a method for the treatment of hyperlipidemia in a mammal.

## Description

### Field of Invention

The current invention relates to the fields of medicinal organic chemistry, pharmacology, and medicine. More particularly, the current invention relates to methods for treating hyperlipidemia in mammals, including humans.

### Background of the Invention

A condition where an abnormally high concentration of lipids circulates in the serum is known as hyperlipidemia. The composition of the lipid pool in the circulation consists mostly of triglyceride (fatty acid esters of glycerol), cholesterol, and fatty acid esters of cholesterol. These molecules are hydrophobic and are poorly soluble in the aqueous environment of the serum. As such, they are generally bound to and are carried by specific proteins, known as apoproteins. Various combinations of different and specific lipids and apoproteins form lipoproteins. Lipoproteins can transport lipids and perform specific biological functions. In general, the lipoproteins are physically classified by their density, e.g., high density lipoproteins (HDL) (1.063-1.210 g/mL), low density lipoproteins (LDL) (1.019-1.063 g/mL), very low density lipoproteins (VLDL) (<1.006 g/mL). In addition, each of these lipoproteins contains a specific profile of lipid composition, e.g., HDL contains mostly cholesterol and its esters, whereas VLDL's contain more or exclusively triglycerides.

Common pathological sequelae of hyperlipidemia are atherosclerosis, hypertension, ischemic events (for example, myocardial infarction, cerebral stroke, and organ insufficiency) and thrombosis. Presently, a clinical index is employed to help identify potential factors which may contribute to a pathological sequelae of hyperlipidemia. One of the factors is the level of fasting triglycerides in the serum. Generally, in adults, total serum triglyceride levels greater than about 400 mg/dL are indicative of potential danger of hyperlipidemia.

Various drugs are available which can lower serum triglycerol levels in human patients. For example, Lopid™ (available from Parke-Davis) , and Tricor™ (available from Abott), are effective in treating Type IV and V hyperlipidemias, with triglyceride levels being abnormally high. However, these drugs may cause many side effects, some of which are quite severe. For example, Lopid™ may cause dyspepsia, abdominal pain, acute appendicitis, atrial fibrilation, gall bladder disease, blurred vision, dizziness and rash; Tricor ™ may cause myopathy, rhabdomyolysis, cholelithiasis, and blood dyscrarias.

There continues to be a need to have improved drugs and methods to treat hyperlipidemias.

### Summary of the Invention

The present invention meets this need and provides for improved methods for treating hyperlipidemias.

In accordance with the present invention, a method for treating hyperlipidemia in a mammal includes a step of administering to the mammal an RAR antagonist and/or an RAR inverse agonist of a retinoid receptor. In one embodiment, the retinoid receptor may be a Retinoic Acid Receptor (RAR). In one embodiment, the RAR may be an RARα, RARβ and/or RARγ.

Further in accordance with the present invention, the method for treating hyperlipidemia includes the step of administering to a mammal, for example a human being, an RAR antagonist or RAR inverse agonist to reduce the mammal's level of circulating cholesterol, fatty acid esters of cholesterol and/or triglyceride.

Any feature or combination of features described herein are included within the scope of the present invention provided that the features included in any such combination are not mutually inconsistent as will be apparent from the context, this specification, and the knowledge of one of ordinary skill in the art.

Additional advantages and aspects of the present invention are apparent in the following detailed description and claims.

### Brief Description of the Drawings

Figure 1 shows the level of serum triglycerides in SJL mice 24 hours after 2 daily dosings of a control, AGN 197116 or AGN 194310.
Figure 2 shows the level of serum triglycerides of SJL male mice after 4 daily oral treatments, followed by 6 hours of fasting before WR-1339 is administered.
Figure 3 shows the level of serum triglycerides of SJL mice 24 hours after two daily oral dosings and 16 hours after one intraperitoneal dosing of AGN 197116.
Figure 4 shows the level of serum triglycerides of SJL mice after oral gavages and intraperitoneal injections of AGN 197116, followed by 6 hours fasting before WR-1339 administration.

### Detailed Description of the Invention

The present invention is, in part, based upon the discovery that an RAR antagonist or an RAR inverse agonist of a retinoid receptor can be administered to a mammal to treat hyperlipidemia.

The vitamin A metabolite retinoic acid has long been recognized to induce a broad spectrum of biological effects. Presently, it is believed that retinoids regulate the activity of two distinct intracellular receptor subfamilies: the Retinoic Acid Receptors (RARs) and the Retinoid X Receptors (RXRs).

The first retinoic acid receptor identified, designated RAR-α, acts to modulate transcription of specific target genes in a manner which is ligand-dependent, as has been shown to be the case for many of the members of the steroid/thyroid hormone intracellular receptor superfamily. The endogenous low-molecular-weight ligand upon which the transcription-modulating activity of RAR-α depends is all-trans-retinoic acid. Retinoic acid receptor-mediated changes in gene expression result in characteristic alterations in cellular phenotype, with consequences in many tissues manifesting the biological response to retinoic acid. Two additional genes closely related to RAR-α are designated as RAR-β and RAR-γ. In the region of the retinoid receptors which can be shown to confer ligand binding, the primary amino acid sequences diverge by less than 15% among the three RAR subtypes or isoforms. All-trans-retinoic acid is a natural ligand for the retinoic acid receptors (RARs) and is capable of binding to these receptors with high affinity, resulting in the regulation of gene expression.

Another member of the steroid/thyroid receptor superfamily was also shown to be responsive to retinoic acid. This new retinoid receptor subtype has been designated Retinoid X Receptor (RXR), because certain earlier data suggested that a derivative of all-trans-retinoic acid may be the endogenous ligand for RXR. Like the RARs, the RXRs are also known to have at least three subtypes or isoforms, namely RXR-α, RXR-β, and RXR-y, with corresponding unique patterns of expression (Manglesdorf et al., Genes & Devel., 6: 329-44 (1992)).

Although both the RARs and RXRs respond to all-trans-retinoic acid in vivo, the receptors differ in several important aspects. First, the RARs and RXRs are significantly divergent in primary structure (e.g., the ligand binding domains of RAR-α and RXR-α have only 27% amino acid identity). These structural differences are reflected in the different relative degrees of responsiveness of RARs and RXRs to various vitamin A metabolites and synthetic retinoids. In addition, distinctly different patterns of tissue distribution are seen for RAR and RXR. For example, in contrast to the RARs, which are not expressed at high levels in the visceral tissues, RXR-α mRNA has been shown to be most abundant in the liver, kidney, lung, muscle and intestine. Finally, the RARs and RXRs have different target gene specificity. For example, response elements have recently been identified in the cellular retinal binding protein type II (CRBPII) and apolipoprotein AI genes which confer responsiveness to RXR, but not RAR. Furthermore, RAR has also been recently shown to repress RXR-mediated activation through the CRBPII RXR response element (Manglesdorf et al., Cell, 66: 555-61 (1991)). These data indicate that two retinoic acid responsive pathways are not simply redundant, but instead manifest a complex interplay.

It is surprisingly discovered that the administration of a composition comprising an RAR antagonist or an RAR inverse agonist to a mammal lowers its lipid concentration, for example circulating lipid concentration. In one embodiment, the administration of an RAR antagonist or an RAR inverse agonist to a mammal, preferably a human being, lowers the level of circulating triglyceride (a lipid) in the mammal.

"Antagonists" are chemical compounds and/or complexes of compounds which are able to bind to the retinoic acid binding site of a retinoid receptor, for example an RAR, thereby blocking the binding of retinoic acid to, and activation of the retinoid receptor.

"Inverse agonists" are chemical compounds and/or complexes of compounds which are able to suppress the basal level of a retinoid receptor, for example an RAR, activity (homo- or heterodimerization and transacting transcriptional control of various genes whose regulation is normally responsive to RAR modulation). A compound will normally be a retinoid receptor antagonist if it is an inverse agonist, but the converse is not necessarily true.

Some examples of structures and methods of making and using preferred retinoid receptor, for example RAR, antagonists and inverse agonists are provided in U.S. Patent No. 5,776,699 and U.S. Patent Applications Serial No. 08/998,319, 08/880,823, and 08/840,040 which are all incorporated by reference herein in their entirety. Many of the following compounds are included in one or more of these applications.

A class of preferred compounds has the structure: 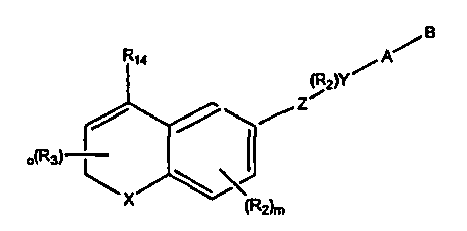 wherein X is S, O, NR' where R' is H or alkyl of 1 to 6 carbons, or
X is [C (R₁)₂]ₙ where R₁ is independently H or alkyl of 1 to 6 carbons, and n is an integer between, and including, 0 and 2, and;
R, is hydrogen, lower alkyl of 1 to 6 carbons, F, Cl, Br, I, CF₃, fluoro substituted alkyl of 1 to 6 carbons, OH, SH, alkoxy of 1 to 6 carbons, or alkylthio of 1 to 6 carbons, and;
R₃ is hydrogen, lower alkyl of 1 to 6 carbons or F, and;
m is an integer having the value of 0 - 3, and;
o is an integer having the value of 0 - 3, and;
Z is -C≡C-,
-N=N-,
-N=CR₁-,
-CR₁=N,
- (CR₁=CR₁)_{n'}- where n' is an integer having the value 0 - 5,
-CO-NR₁-,
-CS-NR₁- ,
-NR₁-CO,
-NR₁-CS,
-COO-,
-OCO-;
-CSO-;
-OCS-;
Y is a phenyl or naphthyl group, or heteroaryl selected from a group consisting of pyridyl, thienyl, furyl, pyridazinyl, pyrimidinyl, pyrazinyl, thiazolyl, oxazolyl, imidazolyl and pyrrazolyl, said phenyl and heteroaryl groups being optionally substituted with one or two R₂ groups, or
when Z is - (CR₁=CR₁)_{n'} - and n' is 3, 4 or 5 then Y represents a direct valence bond between said (CR₂=CR₂)_{n'} group and B;
A is (CH₂)_{q} where q is 0-5, lower branched chain alkyl having 3-6 carbons, cycloalkyl having 3-6 carbons, alkenyl having 2-6 carbons and 1 or 2 double bonds, alkynyl having 2-6 carbons and 1 or 2 triple bonds;
B is hydrogen, COOH or a pharmaceutically acceptable salt thereof, COOR₈, CONR₉R₁₀, -CH₂OH, CH₂OR₁₁, CH₂OCOR₁₁, CHO, CH(OR₁₂)₂, CHOR₁₃O, -COR₇, CR₇(OR₁₂)₂, CR₇OR₁₃O, or tri-lower alkylsilyl, where R, is an alkyl, cycloalkyl or alkenyl group containing 1 to 5 carbons, R₈ is an alkyl group of 1 to 10 carbons or trimethylsilylalkyl where the alkyl group has 1 to 10 carbons, or a cycloalkyl group of 5 to 10 carbons, or R₈, is phenyl or lower alkylphenyl, R₉ and R₁₀ independently are hydrogen, an alkyl group of 1 to 10 carbons, or a cycloalkyl group of 5-10 carbons, or phenyl or lower alkylphenyl, R₁₁ is lower alkyl phenyl or lower alkylphenyl, R₁₂ is lower alkyl, and R₁₃ is divalent alkyl radical of 2-5 carbons, and
R₁₄ is (R₁₅)ᵣ-phenyl, (R₁₅)ᵣ-naphthyl, or (R₁₅)ᵣ-heteroaryl where the heteroaryl group has 1 to 3 heteroatoms selected from the group consisting of O, S and N, r is an integer having the values of 0 - 5, and
R₁₅ is independently H, F, Cl, Br, I, NO₂, N(R₈)₂, N(R₈)COR₈, NR₈CON(R₈)₂, OH, OCOR₈, OR₈, CN, an alkyl group having 1 to 10 carbons, fluoro substituted alkyl group having 1 to 10 carbons, an alkenyl group having 1 to 10 carbons and 1 to 3 double bonds, alkynyl group having 1 to 10 carbons and 1 to 3 triple bonds, or a trialkylsilyl or trialkylsilyloxy group where the alkyl groups independently have 1 to 6 carbons.

Another preferred class of compounds has the structure: 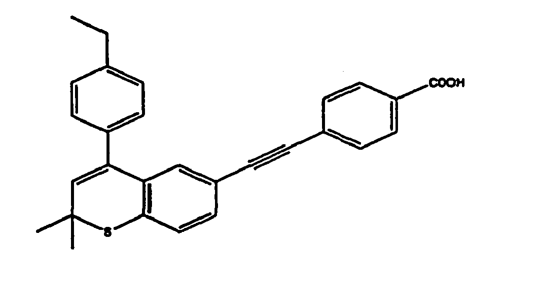 wherein X is S, O, NR' where R' is H or alkyl of 1 to 6 carbons, or
X is [C(R₁)₂]ₙ where R₁ is independently H or alkyl of 1 to 6 carbons, and n is an integer between, and including, 0 and 2, and;
R₂ is hydrogen, lower alkyl of 1 to 6 carbons, F, Cl, Br, I, CF₃, fluoro substituted alkyl of 1 to 6 carbons, OH, SH, alkoxy of 1 to 6 carbons, or alkylthio of 1 to 6 carbons, and;
R₃ is hydrogen, lower alkyl of 1 to 6 carbons or F, and;
m is an integer having the value of 0, 1., 2, or 3, and;
o is an integer having the value of 0, 1, 2, or 3, and;
Y is a phenyl or naphthyl group, or heteroaryl selected from a group consisting of pyridyl, thienyl, furyl, pyridazinyl, pyrimidinyl, pyrazinyl, thiazolyl, oxazolyl, imidazolyl and pyrrazolyl, said phenyl and heteroaryl groups being optionally substituted with one or two R₂ groups, and;
A is (CH₂)_{q} where q is 0-5, lower branched chain alkyl having 3-6 carbons, cycloalkyl having 3-6 carbons, alkenyl having 2-6 carbons and 1 or 2 double bonds, alkynyl having 2-6 carbons and 1 or 2 triple bonds, and;
B is hydrogen, COOH or a pharmaceutically acceptable salt thereof, COOR₈, CONR₉R₁₀, -CH₂OH, CH₂OR₁₁, CH₂OCOR₁₁, CHO, CH(OR₁₂)₂, CHOR₁₃O, -COR₇, CR₇(OR₁₂)₂, CR₇OR₁₃O, or tri-lower alkylsilyl, where R₇ is an alkyl, cycloalkyl or alkenyl group containing 1 to 5 carbons, R₈ is an alkyl group of 1 to 10 carbons or trimethylsilylalkyl where the alkyl group has 1 to 10 carbons, or a cycloalkyl group of 5 to 10 carbons, or R₈ is phenyl or lower alkylphenyl, R₉ and R₁₀ independently are hydrogen, an alkyl group of 1 to 10 carbons, or a cycloalkyl group of 5-10 carbons, or phenyl or lower alkylphenyl, R₁₁ is lower alkyl, phenyl or lower alkylphenyl, R₁₂ is lower alkyl, and R₁₃ is divalent alkyl radical of 2-5 carbons, and;
R₁₄ is (R₁₅)ᵣ-phenyl, (R₁₅)ᵣ-naphthyl, or (R₁₅)ᵣ-heteroaryl where the heteroaryl group has 1 to 3 heteroatoms selected from the group consisting of 0, S and N, r is an integer having the values of 0,1, 2, 3, 4 or 5, and;
R₁₅ is independently H, F, Cl, Br, I, NO₃, N(R₈)₂, N(R₈)COR₈, NR₈CON(R₈)₂, OH, OCOR₈, OR₈, CN, an alkyl group having 1 to 10 carbons, fluoro substituted alkyl group having 1 to 10 carbons, an alkenyl group having 1 to 10 carbons and 1 to 3 double bonds, alkynyl group having 1 to 10 carbons and 1 to 3 triple bonds, or a trialkylsilyl or trialkylsilyloxy group where the alkyl groups independently have 1 to 6 carbons, and;
R₁₆ is H, lower alkyl of 1 to 6 carbons, and;
R₁₇ is H, lower alkyl of 1 to 6 carbons, OH or OCOR₁₁, and;
p is 0 or 1, with the proviso that when p is 1 then there is no R17 substituent group, and m is an integer between, and including, 0 and 2.

A further preferred class of compounds is the class of the structure: 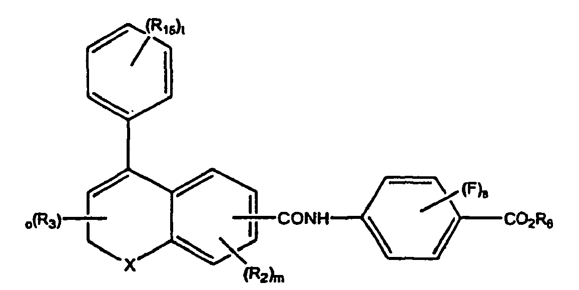 where X is C(R₁)₂ or O, and;
R₁ is H or alkyl of 1 to 6 carbons, and;
R₂ is lower alkyl of 1 to 6 carbons, F, Cl, Br, I, CF_{3'} fluoro substituted alkyl of 1 to 6 carbons, OH, SH, alkoxy of 1 to 6 carbons, or alkylthio of 1 to 6 carbons, and;
m is an integer having the value of 0-3, and;
R₃ is lower alkyl of 1 to 6 carbons of F, and;
o is an integer having the value of 0-3, and;
s is an integer having the value of 1-3, and;
R₈ is an alkyl group of 1 to 10 carbons or trimethylsilylalkyl where the alkyl group has 1 to 10 carbons, or a cycloalkyl group of 5 to 10 carbons, or R₈ is phenyl or lower alkylphenyl, and;
R₁₅ is independently H, F, Cl, Br, I, NO₂, N(R₈)₂, COR₈, NR₈CON(R₈)₂, OCOR₈, OR₈, CN, an alkyl group having 1 to 10 carbons, fluoro substituted alkyl group having 1 to 10 carbons, an alkenyl group having 1 to 10 carbons and 1 to 3 double bonds, an alkynyl group having 1 to 10 carbons and 1 to 3 triple bonds, or a trialkylsilyl or trialkylsilyloxy group where the alkyl groups independently have 1 to 6 carbons, and;
t is an integer having the values of 0, 1, 2, 3, 4, or 5, and;
the CONH group is in the 6 or 7 position of the benzopyran, and in the 2 or 3 position of the dihydronaphthaline ring, or a pharmaceutically acceptable salt of said compound.

Another preferred class of compounds is that of the structure: 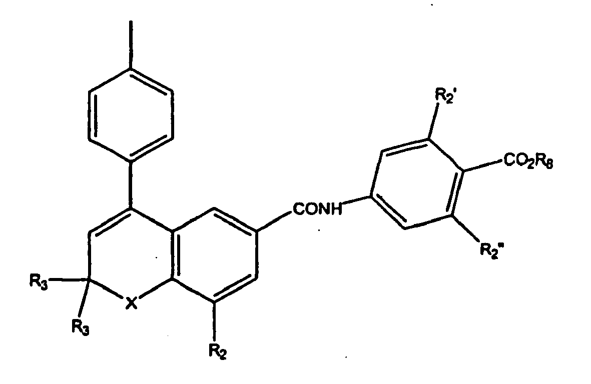 where X is C (CH₃)₂ or O, and;
R₂ is H or Br, and;
R₂, and R₂,, independently are H or F, and;
R₃ is H or CH₃, and;
R₈ is H, lower alkyl of 1 to 6 carbons, or a pharmaceutically acceptable salt of said compound.

A further preferred class of such compounds has the structure: 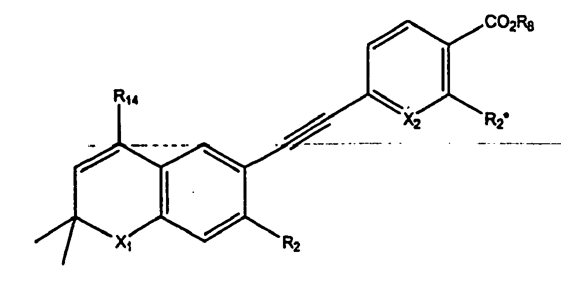 where X₁ is S or O;
X₂ is CH or N;
R₂ is H, F, CF₃ or alkoxy of 1 to 6 carbons;
R₂* H, F, or CF₃;
R₈ is H, or lower alkyl of 1 to 6 carbons;
R₁₄ is unsubstituted phenyl, thienyl or pyridyl, or phenyl, thienyl or pyridyl substituted with one to three R₁₅ groups, where R₁₅ is lower alkyl of 1 to 6 carbons, chlorine, CF_{3'} or alkoxy of 1 to 6 carbons, or a pharmaceutically acceptable salt of said compound.

In yet another preferred embodiment of the invention, the compound has the structure: 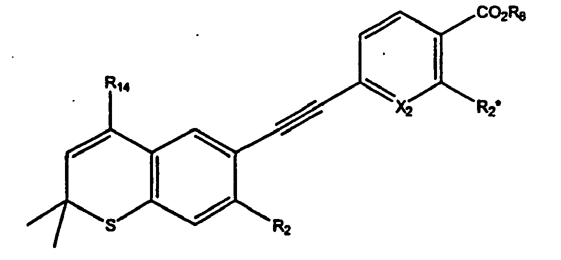 wherein X₂ is CH or N, and;
R₂ is H, F, or OCH₃, and;
R₂* H or F, and;
R₈ is H, or lower alkyl of 1 to 6 carbons, and; R₁₄ is selected from the group consisting of phenyl, 4 - (lower- alkyl) phenyl, 5- (lower alkyl)-2-thienyl, and 6-(lower alkyl)-3-pyridyl where lower alkyl has 1 to 6 carbons, or a pharmaceutically acceptable salt of said compound.

A further preferred class of such compounds has the structure: 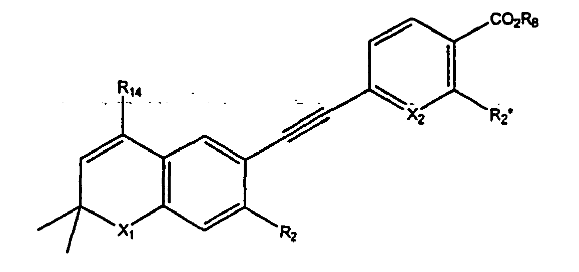 where X₁ is S or 0;
X₂ is CH or N;
R₂ is H, F, CF₃ or alkoxy of 1 to 6 carbons;
R₂* H, F, or CF₃;
R₈ is H, or lower alkyl of 1 to 6 carbons;
R₁₄ is unsubstituted phenyl, thienyl or pyridyl, or phenyl, thienyl or pyridyl substituted with one to three R₁₅ groups, where R₁₅ is lower alkyl of 1 to 6 carbons, chlorine, CF₃, or alkoxy of 1 to 6 carbons, or a pharmaceutically acceptable salt of said compound.

In an even more preferred embodiment of the invention, the compound has the structure: 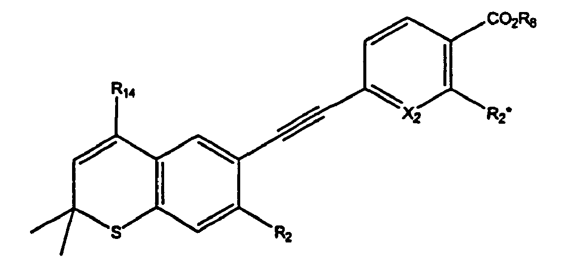 wherein X₂ is CH or N, and;
R₂ is H, F, or OCH₃, and;
R₂* H or F, and;
R₈ is H, or lower alkyl of 1 to 6 carbons, and;
R₁₄ is selected from the group consisting of phenyl, 4-(lower-alkyl)phenyl, 5-(lower alkyl)-2-thienyl, and 6-(lower alkyl)-3-pyridyl where lower alkyl has 1 to 6 carbons, or a pharmaceutically acceptable salt of said compound.

Another class of compounds for use in a preferred embodiment of the present invention has the following structure: 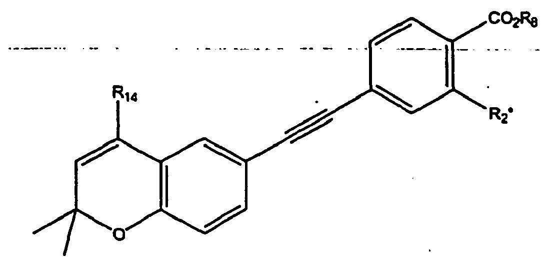 where R₂* is H or F;
R₈ is H, or lower alkyl of 1 to 6 carbons, and
R₁₄ is selected from the group consisting of phenyl, and 4-(lower-alkyl)phenyl, where lower alkyl has 1 to 6 carbons, or a pharmaceutically acceptable salt of said compound.

Another preferred compound class has the following structure: 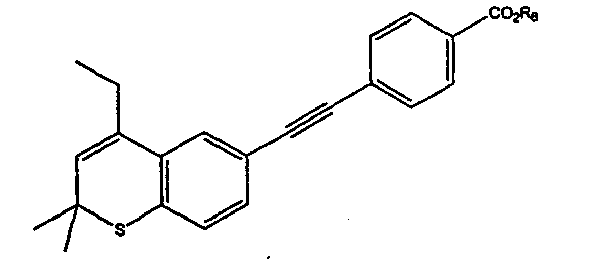 where R₈ is H, lower alkyl of 1 to 6 carbons, or a pharmaceutically acceptable salt of said compound.

Yet another preferred compound is one having the following structure: 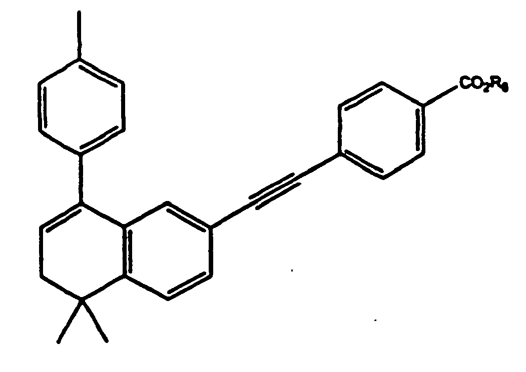 where R₈ is H, lower alkyl of 1 to 6 carbons, or a pharmaceutically acceptable salt of said compound. When R₈ is H, this compound is termed AGN 193109.

Yet another class of compounds contemplated for use in the present invention is that having the structure: 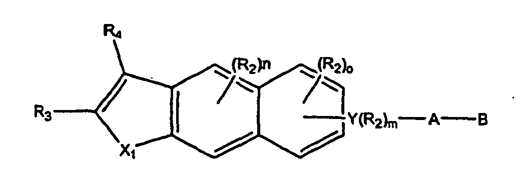 wherein X₁ is: -C(R₁)₂-_{'} -C(R₁)₂-C(R₁)₂-_{'} -S-, -0-, -NR₁- , -C(R₁)₂-O-, -C(R₁)₂-S-, or C(R₁)₂-NR₁-; and
R₁ is independently H or alkyl of 1 to 6 carbons; and
R₂ is optional and is defined as lower alkyl of 1 to 6 carbons, F, Cl, Br, I, CF_{3'} fluoro substituted alkyl of 1 to 6 carbons, OH SH, alkoxy of 1 to 6 carbons, or alkylthio of 1 to 6 carbons; and
m is an integer between, and including, 0 and 4; and
n is an integer between, and including, 0 and 2; and
o is an integer between, and including, 0 and 3; and
R₃ is H, lower alkyl of 1 to 6 carbons, F, Cl, Br or I; and
R₄ is (R₅)ₚ-phenyl , (R₅)ₚ-naphthyl, (R₅)ₚ-heteroaryl where the heteroaryl group is five-membered or 6-membered and has 1 to 3 heteroatoms selected from the group consisting of O, S, and N; and
p is an integer between, and including, 0 and 5; and
R₅ is optional and is defined as independently F, Cl, Br, I, NO₂, N(R₈)₂, N(R₈)COR₈, N(R₈)CON(R₈)₂, OH, OCOR₈, OR₈, CN, COOH, COOR₈, an alkyl group having from 1 to 10 carbons, an alkenyl group having from 1 to 10 carbons and 1 to three double bonds, alkynyl group having from 1 to 10 carbons and 1 to 3 triple bonds, or a (trialkyl)silyl or (trialkyl)silyloxy group where the alkyl groups independently have from 1 to 6 carbons; and
Y is a phenyl or naphthyl group, or a heteroaryl selected from the group consisting of pyridyl, thienyl, furyl, pyridazinyl, pyrimidinyl, pyrazinyl, thiazolyl, oxazolyl, imidazolyl and pyrrazolyl, said phenyl and heteroaryl groups being optionally substituted with one or two R₂ groups, or Y is - (CR₃=CR₃)ᵣ-; and
r is an integer between, and including, 1 and 3; and
A is (CH₂)_{q} where q is an integer from 0-5, lower branched chain alkyl having from 3 to 6 carbons, cycloalkyl having from 3 to 6 carbons, alkenyl having from 2 to 6 carbons and 1 or 2 double bonds, alkenyl having from 2 to 6 carbons and 1 or 2 triple bonds, with the proviso that when Y is - (CR₃=CR₃) ᵣ- then A is (CH₂) _{q} and q is 0; and
B is H, COOH or a pharmaceutically acceptable salt thereof, COOR₈, CONR₉R₁₀, -CH₂OH, CH₂OR₁₁, CH₂OCOR₁₁, CHO, CH (OR₁₂)₂, CHOR₁₃O, -COR₇, CR₇(OR₁₂)₂, CR₇OR₁₃O, or Si (C₁₋₆alkyl)₃, wherein R₇ is an alkyl, cycloalkyl or alkenyl group containing 1 to_5 carbons, R₈ is an alkyl group of 1 to 10 carbons or (trimethylsilyl)alkyl, where the alkyl groups has 1 to 10 carbons, or a cycloalkyl group of 5 to 10 carbons, or R₈ is phenyl or lower alkylphenyl, R₉ and R₁₀ independently are H, a lower alkyl group of 1 to 10 carbons, or a cycloalkyl group of 5-10 carbons, or phenyl or lower alkylphenyl, R₁₁ is lower alkyl, phenyl or lower alkylphenyl, R₁₂ is lower alkyl, and R₁₃ is a divalent alkyl radical of 2-5 carbons. A non-exclusive list of compounds falling within this description, and methods for making this class of compounds are disclosed in U.S. Patent No. 5,728,846 to Vuligonda et al., the disclosure of which is hereby incorporated by reference as part of this application.

Also useful in the present invention are compounds of the formula:

Y₂ (R₄) -X-Y₁ (R₁R₂) -Z-Y₂ (R₂) -A-B

Where Y₁ is phenyl, naphthyl, or heteroaryl selected from the group consisting of pyridyl, thienyl, furyl, ozazolyl, imidazolyl, and pyrrazolyl, said phenyl, naphthyl, and heteroaryl groups being substituted with an R₁ group, and further substituted or unsubstituted with one or two R₂ groups;
R₁ is C₁₋₁₀ alkyl, 1-ademantyl, 2-tetrahydropyranoxy, trialkylsilanyloxy where alkyl has up to 6 carbons, OH, alkoxy where the alkyl group has up to 10 carbons, alkylthio where the alkyl group has up to 10 carbons, or OCH₂OC₁₋₆alkyl;
R₂ is lower alkyl of 1 to 6 carbons, F, Cl, Br, I, CF_{3'} CF₂CF₃, OH, OR₃, NO₂, N(R₃)₂, CN, N₃, COR₃, NHCOR₃, COOH, or COOR₃;
X is (C (R₃)₂, S, SO, SO₂, O or NR₃;
Z is -C≡C-,
-N=N-,
-N (O) =N-,
-N=N(O)-,
-N=CR₃-,
-CR₃=N,
-(CR₃=CR₃)ₙ- where n is an integer having the value 0 - 5,
-CO-NR₃-,
-CS-NR₃-,
-NR₃-CO,
-NR₃-CS,
-COO-,
-OCO-;
-CSO-;
-OCS-; or
-CO-CR₃=R₃-O,
R, is independently H or lower alkyl of 1 to 6 carbons;
Y₂ is a phenyl or naphthyl group, or heteroaryl selected from a group consisting of pyridyl, thienyl, furyl, pyridazinyl, pyrimidinyl, pyrazinyl, thiazolyl, oxazolyl, imidazolyl and pyrrazolyl, said phenyl, naphthyl and heteroaryl groups being unsubstituted or substituted with one or two R₂ groups, or
when Z is -(CR₃=CR₃)ₙ- and n is 3, 4 or 5 then Y₂ represents a direct valence bond between said -(CR₃=CR₃)ₙ group and B;
Y₃ is phenyl, naphthyl, or heteroaryl selected from a group consisting of pyridyl, thienyl, furyl, pyridazinyl, pyrimidinyl, pyrazinyl, thiazolyl, oxazolyl, imidazolyl and pyrrazolyl, said phenyl, naphthyl and heteroaryl groups being unsubstituted or substituted with one to three R₄ groups, where R₄ is alkyl of 1 to 10 carbons, fluoro-substituted alkyl of 1 to 10 carbons, alkenyl of 2 to 10 carbons and having 1 to 3 triple bonds, F, Cl, Br, I, NO₂, CN, NR₃, N₃, COOH, COOC₁₋₆ alkyl, OH, SH, OC₁₋₆ alkyl, and SC₁₋₆ alkyl;
A is (CH₂)_{q} where q is from 0-5, lower branched alkyl having 3-6 carbons, cycloalkyl having 3-6 carbons, alkenyl, having 2-6 carbons and 1-2 double bonds, alkynyl having 2-6 carbons and 1 to 2 triple bonds, and
B is hydrogen, COOH or a pharmaceutically acceptable salt thereof, COOR₈, CONR₉R₁₀, -CH₂OH, CH₂OR₁₁, CH₂OCOR₁₁, CHO, CH (OR₁₂)₂, CHOR₁₃O, -COR₇, CR₇(OR₁₂)₂, CR₇OR₁₃O, or Si(C₁₋₆ alkyl)₃, where R₇ is an alkyl, cycloalkyl or alkenyl group containing 1 to 5 carbons, R₈ is an alkyl group of 1 to 10 carbons or trimethylsilylalkyl where the alkyl group has 1 to 10 carbons, or a cycloalkyl group of 5 to 10 carbons, or R₈ is phenyl or lower alkylphenyl, R₉ and R₁₀ independently are hydrogen, an alkyl group of 1 to 10 carbons, or a cycloalkyl group of 5-10 carbons, or phenyl or lower alkylphenyl, R₁₁ is lower alkyl, phenyl or lower alkylphenyl, R₁₂ is lower alkyl, and R₁₃ is divalent alkyl radical of 2-5 carbons, or a pharmaceutically acceptable salt of said compound. These compounds are disclosed in U.S. Patent Application Serial No. 08/840,040, to Song et al., which application shares common ownership with the present application and is incorporated by reference herein in its entirety.

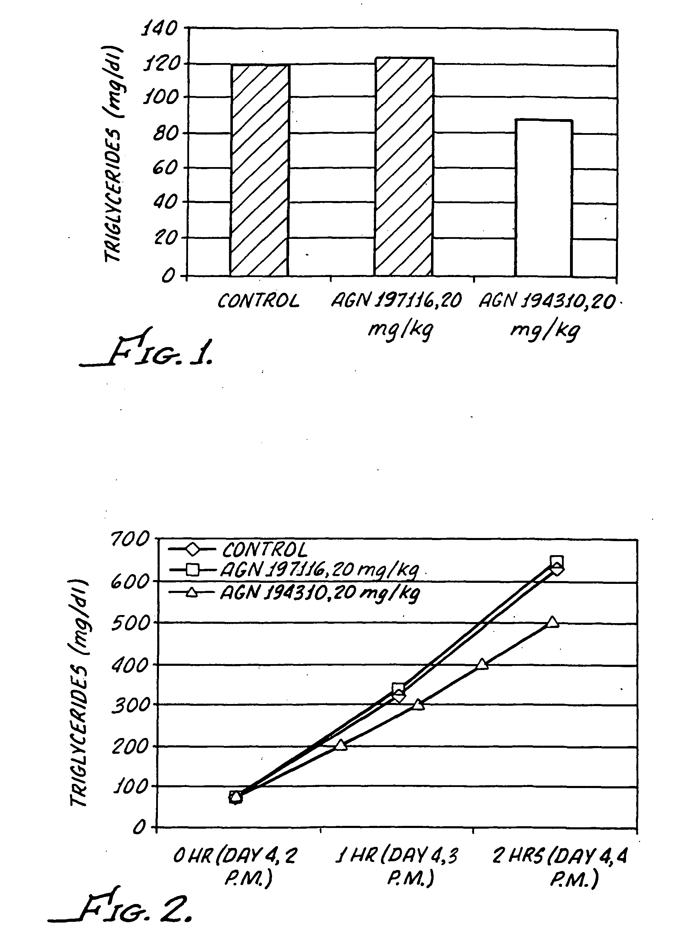

Additional RAR antagonists or inverse agonists 08/845,019, to Song and Chandraratna, which is incorporated by reference herein in its entirety; this application shares common ownership with the present application. Also, compounds useful in the methods of the present invention are disclosed in International Application Publication No. WO 94/14777, to Yoshimura et al., which is also incorporated by reference herein in its entirety. This latter application discloses RAR antagonists. A non-exclusive list of the structures of some preferred compounds disclosed therein can be found in Figure 1 hereof.

Furthermore, the structures of additional compounds useful in the present invention are disclosed below. 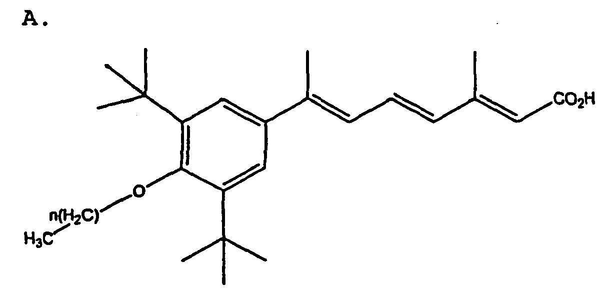 where n is an integer from 1 to 10. 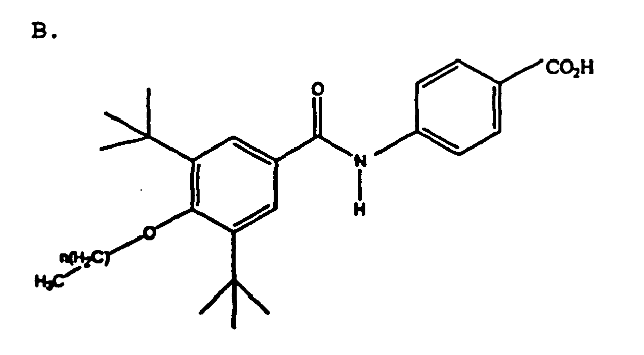 where n is an integer from 1 to 10.

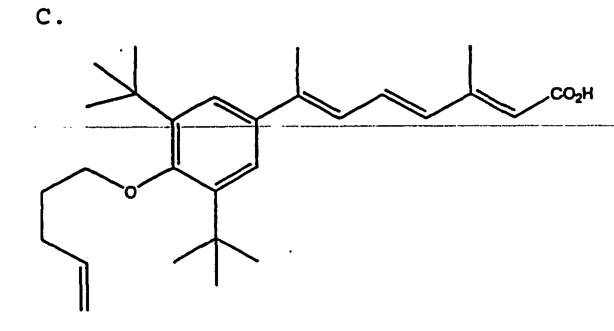

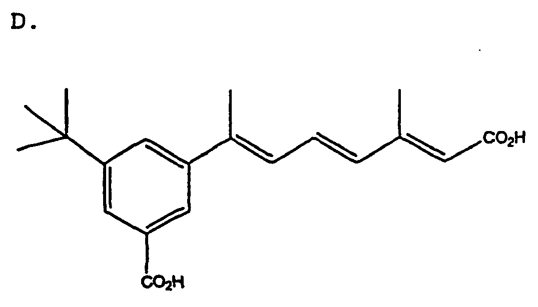

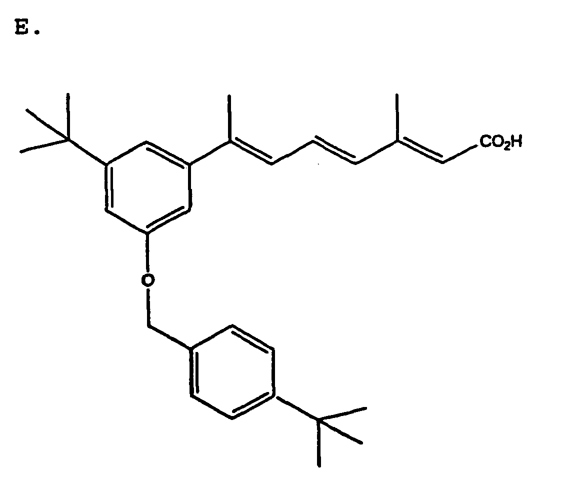

A particularly preferred subgroup of RAR antagonists or inverse agonists is the set of those RAR antagonists or inverse agonists that lack antagonist or inverse agonist activity at one or more subclasses of RARs, such as the RARα, RARβ, or RARγ receptors; such "subclass-specific" activity may result in the minimization of toxicity of the drug. Such compounds may have activity only at the RARα, RARβ, or RARγ receptors, or at any combination of these (other than at all of them). Determination of whether a compound has subclass-specific inverse agonist activity is done through translational screening as disclosed in U.S. Patent Application Serial No. 09/042,943, to Klein et al., and Serial No. 09/108,298, to Nagpal et al., both of which are incorporated by reference herein in their entirety.

The compounds disclosed herein clearly suggest the synthesis and use of other compounds structurally similar to these, for use in the methods of the present invention. In addition to the compounds referred to herein, other compounds that have RAR antagonist and/or inverse agonist activity are also anticipated to lower the level of lipid, preferably triglycerol, and thus be useful in treating hyperlipidemia.

For therapeutic applications in accordance with the present invention the RAR antagonist and RAR inverse agonist compounds may be incorporated into pharmaceutical compositions, such as tablets, pills, capsules, solutions, suspensions, creams, ointments, gels, salves, lotions and the like, using such pharmaceutically acceptable excipients and vehicles which per se are well known in the art. For example, preparation of topical formulations are well described in Remington's Pharmaceutical Science, Edition 17, Mack Publishing Company, Easton, Pa; incorporated by reference herein. For topical application, the RAR antagonist or inverse agonist compounds could also be administered as a powder or spray, particularly in aerosol form. If the RAR antagonist or RAR inverse agonist is to be administered systemically, it may be prepared as a powder, pill, tablet or the like or as a syrup or elixir suitable for oral administration. For intravenous or intraperitoneal administration, the RAR antagonist or RAR inverse agonist may be prepared as a solution or suspension capable of being administered by injection. In certain cases, it may be useful to formulate the antagonist or inverse agonist compounds in a solution for injection. In other cases, it may be useful to formulate the antagonist or inverse agonist compounds in suppository form or as extended release formulation for deposit under the skin or intramuscular injection.

The antagonist or inverse agonist compounds will be administered in a therapeutically effective dose in accordance with the invention. A therapeutic concentration will be that concentration which is effective to lower the concentration of lipids, for example triglycerol, in a mammal, preferably a human being. It is currently thought that a formulation containing between about 0.1 and about 3 mg of an RAR antagonist or inverse agonist/kg of body weight, more preferably between about 0.3 mg/kg and 2 mg/kg, even more preferably about 0.7 mg/kg and about 1.5 mg/kg will constitute a therapeutically effective concentration for oral application, with routine experimentation providing adjustments to these concentrations for other routes of administration if necessary.

In a further preferred embodiment, a pharmaceutical composition comprising the RAR antagonist or RAR inverse agonist is administered orally. Such composition may be in the form of a liquid, syrup, suspension, tablet, capsule, or gelatin-coated formulation. In another preferred embodiment, a pharmaceutical composition comprising an RAR antagonist or RAR inverse agonist is topically administered. Such composition may be in the form of a patch, cream, lotion, emulsion, or gel. In yet another embodiment, a pharmaceutical composition comprising the RAR antagonist or RAR inverse agonist may be inhaled. Such composition may be formulated as an inhalant, suppository or nasal spray.

The following examples are intended to illustrate further embodiments of the present invention and do not limit the scope of the invention, which is defined solely by the claims concluding this specification.

### Example 1

A 32-year-old, obese, Caucasian male has a cholesterol level of 299 g/mL, a triglyceride level of 440mg/dL, an LDL level of 199 g/mL, and an HDL level of 25g/mL. He does not have diabetes, kidney, or liver disease. He has a family history of coronary artery disease--his father suffers a heart attack at age 50.

Because this patient is a male, obese, and has a positive family history of heart disease, he is advised to immediately start using the composition of the present invention on a daily basis. Preferably, the composition is a tablet containing 20 mg of AGN 194310. Additionally, he must strictly adhere to a low fat diet, and regularly exercise 30 minutes daily or 45 minutes every other day.

The patient follows up with his doctor in 3 months with a repeat lipid profile. The blood test result shows an improvement of decreased cholesterol and triglycerides to 250 g/mL and 280 mg/dL, respectively. The follow up plan also includes maintaining the same dosage of composition at 20 mg for two months, since the patient tolerates the medication well.

### Example 2

A 45-year-old Hispanic male with a history of gout and gastritis has a triglyceride level of 950 mg/dL, and a cholesterol level of 300 g/mL. The patient begins using the composition of the present invention, for example a tablet containing 50 mg of AGN 194320, twice daily with no side effects. The patient is very compliant with respect to taking the medication everyday, along with consuming a low fat diet and regularly exercising. As a result, the patient's triglyceride level decreases to 450 mg/dL. His gout and gastritis conditions also improve as a direct result of lowering his triglycerides levels and his low fat diet. He is to maintain the dosage of a composition of the present invention at 50mg twice daily for the best results.

### Example 3

A 55-year-old Asian female has menopause, hypertension, and hyperlipidemia. She is currently taking Prempro™ hormone replacement therapy for menopause, and Atenolol™ for hypertension, which is controlled at this time. Her lipid profiles show an elevated LDL level of 180 g/mL (normal < 130), a low HDL level of 28 g/mL (normal> 40), a normal triglyceride level of 170 mg/dL (normal <160), and a cholesterol level of 210 g/mL (normal < or = 200).

Since the patient does not like to take medication, her doctor agrees to wait six to twelve months to monitor her lipid profiles without the lipid-lowering medication, counting on the hormone replacement therapy and a low fat diet to help reduce the LDL cholesterol level. However, after one year, the LDL and HDL levels are not adequately reduced. Her doctor decides to start administering a composition of the present invention at a dose of 10 mg daily for 6 months. Subsequently, the LDL level decreased to 130 g/mL and the HDL level increased to 60 g/mL. Even though the patient's lipid profile improved to normal range, it is recommended that she continues to take the composition of the present invention, for example a tablet containing 10 mg of AGN 194310 daily, to prevent future accumulation of LDL, which causes cholesterol plague in coronary vessels. Also, she is recommended to take 81 mg of aspirin daily to prevent stroke and heart disease.

### Example 4

A 34-year-old Hispanic female with diabetes mellitus type 2 has high cholesterol levels and high LDL levels. During an office visit, she experiences a silent heart attack without congestive heart failure. She is then admitted to the hospital for further cardiac evaluation and subsequently discharged after three days. She is currently taking Glucotrol™ XL 5mg daily, Glucophage™ 500mg twice a day (diabetes medications), Tenormin™ 25 mg/ day, Zestril™ 10mg/day (to prevent chest pain, and high blood pressure), and aspirin 81mg/day. She is also taking a composition of the present invention at the dosage of 10mg-20mg AGN 194310 daily to prevent a second myocardial infarction in the future.

### Example 5

A 42-year-old Asian male has strong a familial hypercholesterolemia. Hypercholesterolemia is a condition in which cholesterol is overly produced by the liver for unknown reasons. Furthermore, hypercholesterolemia is a strong risk factor for myocardial infarction (MI), diabetes, obesity, and other illnesses. The patient is not overweight, but is very thin. He has a very high level of cholesterol, over 300 g/mL, and a triglyceride level of over 600 mg/dL. His diet consists of very low fat, high protein foods, and no alcohol. He has a very active lifestyle, but one which is not stressful. However, he still has to take medication to lower his cholesterol and triglyceride levels. The medications he takes include a composition of this invention. He is advised to continue taking the composition of this invention, for example a tablet containing 40mg of AGN 194310, daily for the remainder of his life in order to control his unusual familial hypercholesterolemia condition.

### Example 6

A 22-year-old male patient presents with triglyceride level of 250mg/dL. The patient is given oral tablets containing about 20mg to about 100mg of RAR antagonists or inverse agonist, preferably AGN 194310. The patient's level of triglyceride is measured 24 hours after ingesting said tablets. The measurement shows a decrease of about 20% to 50% of triglycerides as compared to the initial level.

### Example 7

Five male cynomologus monkeys were employed in Study PT-99-10. Three of the five monkeys were treated with AGN 194310 at a daily dosage of 1.25 mg/kg (orally) for a period of 25 days. AGN 194310 is an RAR antagonist or inverse agonist. Its structure is described herein below. The remaining two were similarly treated with a vehicle to serve as control. Serum samples were collected on days 1, 8, 15, 22 and 25 for triglyceride determination. Serum samples from days 8, 15, 22 and 25 were also assayed for the concentration of AGN 194310.

All monkeys appeared healthy throughout the study period with no change in body weight or rate of food consumption.

A highly significant decrease of serum triglycerides was observed in each of the three monkeys receiving AGN 194310 treatment (See Table 1). When compared to day 1 (baseline), the average decrease was 52%, 54% and 51% for the three monkeys treated with AGN 194310, while the two control monkeys had an average increase of 48% and 89%.

The triglyceride lowering effect and the relatively high blood concentration of AGN 194310 (Table 2) indicated that AGN 194310 was well absorbed by monkeys when given orally.

From the data presented, it is concluded that AGN 194310 lowers serum triglycerides in monkeys at a daily dose of 1.25 mg/kg without any noticeable abnormal clinical signs.

**Table 1**

| Serum triglycerides (mg/dl) of male cynomolgus monkeys treated with AGN 194310 by gastric intubation. | | | | | | |
|---|---|---|---|---|---|---|
| AGN 194310 | Animal # | Day 1 | Day 8 | Day 15 | Day 22 | Day 25 |
| 0.0mg/0.4ml/kg | 18-18 | 45.1 | 82.2 | 92.1 | 83.8 | 82.9 |
| | 18-40 | 40.7 | 43.5 | 47.8 | 83.6 | 65.4 |
| | Mean | 42.9 | 62.9 | 70.0 | 83.7 | 74.2 |
| 1.0mg/0.4ml/kg | 28-199 | 48.8 | 24.3 | 18.2 | 30.4 | 20.3 |
| | 28-312 | 52.5 | 21.6 | 30.7 | 20.6 | 23.4 |
| | 28-318 | 58.5 | 19.2 | 29.6 | 36.5 | 28.3 |
| | Mean | 53.3 | 21.7 | 26.2 | 29.2 | 24.0 |

**Table 2**

| | | | | | |
|---|---|---|---|---|---|
| Serum concentration (ng/mL) of AGN 194310 in male cynomolgus monkeys treated with AGN 194310 by gastric intubation. | | | | | |

| AGN 194310 | Animal # | Day 8 | Day 15 | Day 22 | Day 25 |
|---|---|---|---|---|---|
| 0.0mg/0.4ml/kg | 18-18 | BLQ | 0.615 | 0.247 | 1.23 |
| | 18-40 | 0.384 | 1.5 | 0.107 | 1.23 |
| 1.0mg/0.4ml/kg | 28-199 | >194 | 1408 | 488 | >2878 |
| | 28-312 | 401 | 140 | 882 | 431 |
| | 28-318 | >148 | >177 | >118 | >1955 |

### Example 8: Effect of RAR antagonists on serum triglycerides and hepatic triglyceride output in male SJL mice.

Male SJL mice were dosed orally with vehicle, AGN 197116 (RARα antagonist) or AGN 194310 (RAR panantagonist) for 4 consecutive days. The structure of AGN 194310 is described herein below. The structure of AGN 197116 is:

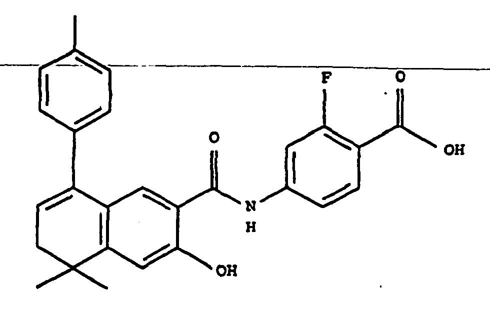

The test compounds were dissolved in corn oil and given at a dosage/volume of 20mg/5ml/kg.

On day 3, serum triglycerides (STG) were determined from samples collected at 7 a.m.

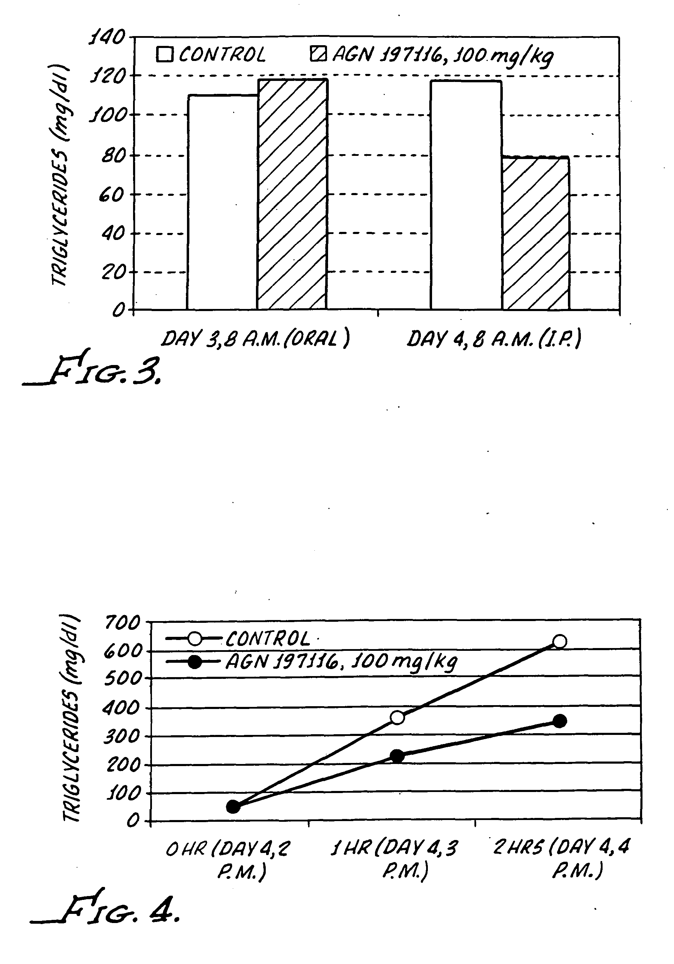

On day 4, animals were fasted after dosing, starting at 8 a.m. Following 6 hours of fasting, blood samples were collected prior to intravenous injection of WR-1339 at 100mg/5ml/kg. Additional serum samples were collected at 1 and 2 hours after WR-1339 injection. WR-1339 is a detergent, which inactivates lipoprotein lipase and thus prevents the removal of triglycerides from circulation. By measuring the increase of STG after WR-1339 administration in fasted animals, one can estimate the hepatic triglyceride (HTG) output during fasting. Results are listed in Table 3 and Figures 1 and 2.

AGN 914310 appeared to lower non-fasting STG (Day 3, 8 a.m.) but not fasting STG (Day 4, 2 p.m.). A reduction of HTG output after WR-1339 injection was observed with AGN 194310. These effects were not observed with AGN 197116 given orally.

The result also indicated that male SJL mouse is a suitable model for in *vivo* screening of retinoid effect on serum triglycerides. The effect could be detected after 2 days of dosing.

Due to the lack of effect of AGN 197116 at 20 mg/kg, the dose was increased to 100mg/kg in the same set of mice. STG was determined on day 3 prior to dosing (Day 3, 8 a.m.). Again, no lowering of STG was observed (Table 3). To ensure that AGN 197116 would be bioavailable, AGN 197116 was dissolved in DMSO and given by intraperitoneal injections, once at 4 p.m. on day 3 and once at 8 a.m. on day 4, at a dosage of 100 mg/kg/injection. Administration of WR-1339 and blood collections on day 4 were similarly conducted as described above. Results (Table 4 and Figures 3 and 4) indicated that a clear lowering of STG was observed 16 hours after a single intraperitoneal 100 mg/kg dose (Day 4, 8 a.m.). Similar to AGN 194310, this effect disappeared after fasting (Day 4, 2 p.m.). HTG output was also reduced with intraperitoneal injection of AGN. 197116. It is likely that AGN 197116 may not be bioavailable when given orally to mice.

Without wishing to limit the invention to any theory or mechanism of operation, it is believed that RAR antagonists are capable of lowering serum triglycerides in mice when they were made bioavailable by proper route of administration. Furthermore, this lowering of triglycerides of RAR antagonists may be due, at least partially, to a reduced HTG output.

**Table 3 Serum triglycerides in mice treated with AGN 197116 by oral gavages.**

| | | Day 3 | Day 4 post-WR-1339 | | |
|---|---|---|---|---|---|
| Group/Treament | Animal // | 8 a.m. | 0 hr (2 pm) | 1 hr (3 p.m.) | 2 hr (4 p.m.) |
| 1 (Males) | 1 | 111.8 | 81.3 | 431.2 | 763.1 |
| Vehicle (corn oil) | 2 | 199.7 | 95.4 | 432.4 | 956.2 |
| 100mg/kg tyloxapol IV | 3 | 154.4 | 75.3 | 468 | 890.3 |
| | 4 | 104.4 | 85.7 | 287.1 | 497 |
| | 5 | 127.4 | 77.6 | 307.8 | 579 |
| | 6 | 133.4 | 73.4 | 226.4 | 391.8 |
| | 7 | 90.8 | 72.7 | 245.2 | 498.3 |
| | 8 | 111.8 | 85 | 289.7 | 523.5 |
| | 9 | 70.6 | 35.9 | 277.5 | 531.2 |
| | 10 | 99.6 | 79.9 | 333 | 679.8 |
| *Group 1 Mean* | | *120.4* | *76.2* | *329.8* | *631.0* |
| *Group 1 SD* | | *36.3* | *15.7* | *84.6* | *185.5* |
| 2 (Males) | 11 | 128.7 | 63.1 | 360.1 | 726.9 |
| 20mg/kg AGN 197116 | 12 | | | | |
| 100mg/kg tyloxapol IV | 13 | 124 | 91.7 | 380.1 | 723.7 |
| | 14 | 150.3 | 43 | 464.1 | 770.2 |
| | 15 | 110.5 | 72.1 | 241.9 | 590 |
| | 16 | 118.6 | 90.8 | 331.7 | 575.2 |
| | 17 | 124.7 | 76 | 329.8 | 700.4 |
| | 18 | 112.5 | 68.2 | 262.6 | 462.8 |
| | 19 | 106.4 | 73.4 | 311 | 659.1 |
| | 20 | 131.4 | 73.4 | 326.5 | 612.6 |
| *Group 2 Mean* | | *123.0* | *72.4* | *334.2* | *646.8* |
| *Group 2 SD* | | *13.3* | *14.6* | *65.1* | *96.2* |
| 3 (Males) | 21 | 71.2 | 76.6 | 216.8 | 328.5 |
| 20mg/kg AGN 194310 | 22 | 105.7 | 76 | | |
| 100mg/kg tyloxapol IV | 23 | 67.9 | 57.3 | 307.2 | 548 |
| | 24 | 113.2 | 74.7 | 294.9 | 562.9 |
| | 25 | 134.8 | 80.5 | 311.7 | 577.1 |
| | 26 | 76.6 | 71.5 | 238.7 | 493.8 |
| | 27 | 63.1 | 73.4 | 303.9 | 508 |
| | 28 | 84.1 | 61.1 | 260 | 550 |
| | 29 | 95.6 | 67.6 | 252.3 | 542.9 |
| | 30 | 115.2 | 76 | 210.9 | 259.4 |
| *Group 3 Mean* | | *92.7* | *71.5* | *266.3* | *485.6* |
| *Croup 3 SD* | | *24.0* | *7.4* | *39.5* | *113.0* |

**Table 4 Serum triglycerides in mice treated with AGN 197116 by oral gavages (day 1 to 3) and subcutaneous injections (day 3 to 4).**

| | | Day 3 | Day 4 | Day 4 post-WR-1339 | | |
|---|---|---|---|---|---|---|
| Group/Treatment | I.D. | 0 Hour | 0hr(8am) | 0 hr (2 pm) | 1hr (3 p.m.) | 2 hr (4 p.m.) |
| 1 | 1 | 167 | 121 | 58 | 527 | 857 |
| Vehicle | 2 | 91 | 112 | 45 | 403 | 695 |
| | 3 | 95 | 140 | 50 | 279 | 544 |
| | 4 | 67 | 51 | 45 | 222 | 415 |
| | 5 | 127 | 160 | 58 | 354 | 585 |
| *Group 1 Mean* | | *109* | *117* | *51* | *357* | *619* |
| *Group 1 SD* | | *39* | *41* | *7* | *118* | *166* |
| 2 | 6 | 81 | 58 | 42 | 220 | 285 |
| AGN 197116 | 7 | 104 | 79 | 36 | 195 | 272 |
| Day 1-3, 100 mg/kg, oral | 8 | 103 | 51 | 42 | 248 | 396 |
| Day 3-4, 100 mg/kg, I.P. | 9 | 139 | 114 | 73 | 345 | 531 |
| | 10 | 107 | 50 | 59 | 126 | 200 |
| | 11 | 171 | 125 | 50 | 197 | 387 |
| *Group 2 Mean* | | *118* | *79* | *50* | *222* | *345* |
| *Group 2 SD* | | *32* | *3*3 | *14* | *72* | *118* |

### Example 9: Synthesis of AGN 194310

AGN 194310 has the following chemical structure: 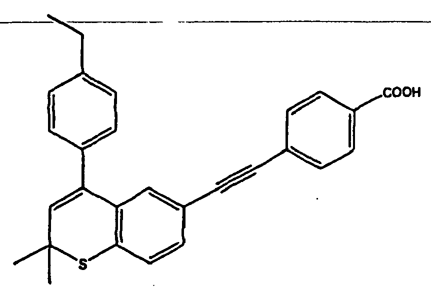 This compound, 4-[[4-(4-ethylphenyl)-2,2-dimethyl-(2H)-thiochromen-6-yl]-ethynyl]-benzoic acid, may be synthesized using conventional organic synthetic means. The following reaction scheme is Applicants' currently preferred method of making this compound.

Step 1: A heavy-walled screw cap tube was charged with 3-methyl-2-butenoic acid (13.86g, 138.4 mmol), 4-methoxy thiophenol (20.0g, 138.4 mmol), and piperidine (3.45 g, 41.6 mmol). This mixture was heated to 105° C for 32 hours, cooled to room temperature and dissolved in EtOAc (700mL). The resulting solution was washed with 1M aqueous HCl, H₂O, and saturated aqueous NaCl before being dried over Na₂SO₄. Concentration of the dry solution under reduced pressure afforded an oil which upon standing in the freezer provided a crystalline solid. 3-(4-methoxy-phenylsulfanyl)-3-methyl-butyric acid was isolated as pale-yellow crystals by washing the crystalline solid with pentane. (27.33 g, 82%). ¹H NMR (300 MHz, CDCl₃) δ: 7.48 (2H, d, J = 9.0 Hz), 6.89 (2H, d, J = 8.9 Hz), 3.83 (3H, s), 2.54 (2H, s), 1.40 (6H, s).

Step 2: To a solution of 3-(4-methoxy-phenylsulfanyl)-3-methyl-butyric acid (20.0 g, 83.2 mmol) in 250 mL of benzene at room temperature was added a solution of oxalyl chloride (15.84g, 124.8 mmol) in 10 mL of benzene over 30 minutes. After 4 hours the solution was washed with ice cold 5% aqueous NaOH (CAUTION: a large volume of gas is released during this procedure), followed by ice cold H₂O, and finally saturated aqueous NaCl. The solution was dried (Na₂SO₄) and concentrated under reduced pressure to give a clear yellow oil. This material was used without further purification in the next step. ¹H NMR (300 MHz, CDCl₃) δ: 7.45 (2H, d, J = 8.8 Hz), 6.90 (2H, d, J = 8.8 Hz), 3.84 (3H, s), 3.12 (2H, s), 1.41 (6H, s). Step 3: To a solution of the acyl chloride product of Step 2 (21.5g, 83.2 mmol) in 250 mL of CH₂Cl₂ at 0° C was added dropwise to a solution of SnCl₄ (21.7g, 83.2 mmol) in 30 mL of CH₂Cl₂. After 2 hours the reaction was quenched by slow addition of 150 mL H₂O. The organic layer was washed with 1M aqueous HCl, 5% aqueous NaOH, H₂O, and finally saturated aqueous NaCl before being dried over MgSO₄. Concentration under reduced pressure and vacuum distillation of the residual oil (Bulb-to-bulb, 125-135° C, 5 mm/Hg) afforded 14.48 g (78%) of 6-methoxy-2,2-dimethyl-thiochroman-4-one as a pale-yellow oil. ¹H NMR (300 MHz, CDCl₃) δ: 7.62 (1H, d, J = 2.9 Hz), 7.14 (1H, d, J = 8.6 Hz), 7.03 (1H, dd, J = 2.8, 8.3 Hz), 3.83 (3H, s), 2.87 (2H, s), 1.46 (6H, s).

Step 4: To a solution of 6-methoxy-2,2-dimethyl-thiochroman-4-one (6.0 g, 27 mmol) in 50 mL CH₂Cl₂ cooled to -23° C was added BBr₃ (20.0 g, 80.0 mmol; 80.0 mL of a 1M solution in CH₂Cl₂) over a 20 minute period. After stirring for 5 hours at -23° C the solution was cooled to -78° C and quenched by the slow addition of 50 mL of H₂O. Upon warming to room temperature the aqueous layer was extracted with CH₂Cl₂ and the combined organic layers were washed with saturated aqueous NaHCO₃, H₂O, and saturated aqueous NaCl before being dried over MgSO₄. Removal of the solvents under reduced pressure gave a green-brown solid, which upon recrystalization (Et₂O / hexanes) afforded 2.25 g (40%) of 6-hydroxy-2,2-dimethylthiochroman-4-one as a light brown solid. ¹H NMR (300 MHz, CDCl₃) δ:7.63 (1H, d, J = 2.8 Hz), 7.15 (1H, d, J = 8.5 Hz), 7.01 (1H, dd, J = 2.8, 8.5 Hz), 2.87 (2H, s), 1.46 (6H, s).

Step 5: To a solution of 6-hydroxy-2,2-dimethylthiochroman-4-one (165.0 mg, 0.79 mmol) in 5.0 mL of anhydrous pyridine at 0° C was added trifluoromethanesulfonic anhydride (245.0 mg, 0.87 mmol). After 4 hours at 0° C the solution was concentrated and the residual oil dissolved in Et₂O, washed with H₂O followed by saturated aqueous NaCl, and dried over MgSO₄. Removal of the solvents under reduced pressure and column chromatography (5% EtOAc / hexanes) afforded 126.0 mg (47%) of 2,2-Dimethyl-4-oxo-thiochroman-6-yl trifluoromethanesulfonate as a colorless solid. ¹H NMR (300 MHz, CDCl₃) δ: 7.97 (1H, s), 7.32 (2H, s), 2.90 (2H, s), 1.49 (6H, s).

Step 6: A solution of 2,2-dimethyl-4-oxo-thiochroman-6-yl trifluoromethanesulfonate (2.88 g, 8.50 mmol) in 10 mL Et₃N and 20.0 mL DMF was sparged with argon for 10 minutes. To this solution was added trimethylsilylacetylene (4.15 g, 42.0 mmol) and bis(triphenylphosphine)-palladium(II) chloride (298.0 mg, 0.425 mmol). The solution was heated to 95° C for 5 hours, cooled to room temperature, and diluted with H₂O. Extraction with EtOAc was followed by washing the combined organic layers with H₂O and saturated aqueous NaCl and drying over MgSO₄. Concentration of the dry solution under reduced pressure and isolation of the product by column chromatography (3% EtOAc / hexanes) afforded 2.23 g (91%) of the 2,2-dimethyl-6-trimethylsilanylethynyl-thiochroman-4-one as an orange oil. ¹H NMR (300 MHz, CDCl₃) δ: 8.18 (1H, d, J = 1.9 Hz), 7.34 (1H, dd, J = 1.9, 8.1 Hz), 7.15 (1H, d, J = 8.1 Hz), 2.85 (2H, s), 1.45 (6H, s), 0.23 (9H, s).

Step 7: A solution of 2,2-dimethyl-6-trimethylsilanylethynyl-thiochroman-4-one (110.0 mg, 0.38 mmol) and K₂CO₃ (40.0 mg, 0.29 mmol) in 10.0 mL MeOH was stirred overnight at room temperature. The solution was diluted with H₂O and extracted with Et₂O. The combined organic layers were washed with H₂O and saturated aqueous NaCl and dried over MgSO₄. Removal of the solvent under reduced pressure afforded 81 mg (99%) of the 6-ethynyl-2,2-dimethylthiochroman-4-one as an orange oil. ¹H NMR (300 MHz, CDCl₃) δ:8.20 (1H, d, J = 1.9 Hz), 7.46 (1H, dd, J = 1.9, 8.1 Hz), 7.18 (1H, d, J = 8.1 Hz), 3.08 (1H, s), 2.86 (2H, s), 1.46 (6H, s).

Step 8: A solution of 6-ethynyl-2,2-dimethylthiochroman-4-one (82.0 mg, 0.38 mmol) and ethyl 4-iodobenzoate (104.9 mg, 0.38 mmol) in 5.0 mL Et₃N was purged with argon for 10 minutes. To this solution were added bis(triphenylphosphine)-palladium(II) chloride (88.0 mg, 0.12 mmol) and copper(I) iodide (22.9 mg, 0.12 mmol). After sparging for an additional 5 minutes with argon, the solution was stirred overnight at room temperature. The reaction mixture was filtered through a pad of Celite using an Et₂O wash. Concentration of the filtrate under reduced pressure, followed by column chromatography of the residual solid, afforded 100 mg (72%) of ethyl 4-[(2,2-dimethyl-4-oxo-thiochroman-6-yl)ethynyl]-benzoate as a yellow solid. ¹H NMR (300 MHz, CDCl₃) δ: 8.25 (1H, d, J = 1.8 Hz), 8.00 (2H, d, J = 8.4 Hz), 7.55 (2H, d, J = 8.4 Hz), 7.53 (1H, dd, J = 1.8, 8.2 Hz), 7.21 (1H, d, J = 8.2 Hz), 4.37 (2H, q, J = 7.1 Hz), 2.88 (2H, s), 1.47 (6H, s), 1.39 (3H, t, J = 7.1 Hz).

Step 9: A solution of sodium bis(trimethylsilyl)amide (1.12 g, 6.13 mmol) in 16.2 mL of THF was cooled to -78° C and a solution of ethyl 4-(2,2-dimethyl-4-oxo-thiochroman-6-ylethynyl)-benzoate (1.86g, 5.10 mmol) in 15.0 mL was added slowly. After 30 minutes a solution of 2-[N,N-bis (trifluoromethanesulfonyl) amino] -5-pyridine (2.40 g, 6.13 mmol) in 10 mL of THF was added. After 5 minutes the solution was warmed to room temperature and stirred overnight. The reaction was quenched by the addition of saturated aqueous NH₄Cl and extracted with EtOAc. The combined organic layers were washed with 5% aqueous NaOH and H₂O before being dried (MgSO₄) and concentrated under reduced pressure. Ethyl 4-((2,2-dimethyl-4-trifluoromethanesulfonyloxy-(2H)-thiochromen-6-yl)ethynyl)-benzoate, 1.53 g (61%), was isolated by column chromatography (2% EtOAc / hexanes) as a yellow solid. ¹H NMR (300 MHz, CDCl₃) δ: 8.03 (2H, d, J = 8.4 Hz), 7.61 (1H, d, J = 1.8 Hz), 7.59 (2H, d, J = 8.4 Hz), 7.41 (1H, dd, J = 1.8, 8.1Hz) , 7.29 (1H, d, J = 8.1 Hz), 5.91 (1H, s), 4.39 (2H, q, J = 7.1 Hz), 1.53 (6H, s), 1.41 (3H, t, J = 7.1 Hz).

Step 10: A solution of 4-ethylbromobenzene (670.9 mg, 3.63 mmol) in 4.0 mL of THF was cooled to - 78° C and *tert-*butyllithium (464.5 mg, 7.25 mmol, 4.26 mL of a 1.7M solution in pentane) was added to give a yellow solution. After 30 minutes a solution of ZnCl₂ (658.7 mg, 4.83 mmol) in 8.0 mL THF was slowly added via cannula. The resulting solution was warmed to room temperature and transferred via cannula to a solution of ethyl 4-(2,2-dimethyl-4-trifluoromethanesulfonyloxy-(2H)-thiochromen-6-ylethynyl)-benzoate (1.20 g, 2.42 mmol) and tetrakis(triphenylphosphine)palladium(0) (111.7 mg, 0.097 mmol) in 8.0 mL THF. This solution was heated to 50° C for 1 hour, cooled to room temperature, and the reaction quenched by the addition of saturated aqueous NH₄Cl. The solution was extracted with EtOAc and the combined organic layers were washed with H₂O and saturated aqueous NaCl before being dried (MgSO₄) and concentrated under reduced pressure. Ethyl 4-((4-(4-ethylphenyl)-2,2-dimethyl-(2H)-thiochromen-6-yl]-ethynyl]-benzoate was isolated by column chromatography (5% EtOAc / hexanes) as a colorless oil. ¹H NMR (300 MHz, CDCl₃) δ: 7.99 (2H, d, *J* = 8.2 Hz), 7.52 (2H, d, J = 8.4 Hz), 7.40 (5H, m), 7.35 (2H, m), 5.85 (1H, s), 4.38 (2H, q, J = 7.1 Hz), 2.72 (2H, q, J = 7.6 Hz), 1.48 (6H, s), 1.40 (3H, t, J = 7.1 Hz) , 1.30 (3H, t, J = 7.6 Hz) .

Step 11: To a solution of ethyl 4-[[4-(4-ethylphenyl)-2,2-dimethyl-(2H)-thiochromen-6-yl]-ethynyl]-benzoate (940.0 mg, 2.08 mmol) in 10.0 mL THF and 5.0 mL EtOH was added NaOH (416.0 mg, 10.4 mmol, 5.2 mL of a 2M aqueous solution). The resulting solution was stirred overnight at room temperature. The reaction mixture was acidified with 10% aqueous HCl and extracted with EtOAc. The combined organic layers were washed with H₂O, saturated aqueous NaCl, and dried (Na₂SO₄) before removing the solvent under reduced pressure. The residual solid was recrystallized from CH₃CN to give 786.0 mg (89%) of 4-[[4-(4-ethylphenyl)-2,2-dimethyl-(2H)-thiochromen-6-yl]-ethynyl]-benzoic acid as a colorless solid. ¹H NMR (300 MHz, d₆-acetone) δ: 8.01 (2H, d, J = 8.3 Hz), 7.60 (2H, d, J = 8.5 Hz), 7.42 (2H, m) , 7.29 (2H, m), 7.22 (3H, m), 5.94 (1H, s), 2.69 (2H, q, J = 7.7 Hz), 1.47 (6H, s), 1.25 (3H, t, J = 7.7 Hz). This compound, the final desired product, was termed AGN 194310.

The AGN 194310 compound was provided as follows: the compound was dissolved in capric/caprylic triglyceride (CCT) at a variety of doses, either 0.001% (v/v) AGN 194310, 0.003% (v/v) AGN 194310, or 0.01% (v/v) AGN 194310. Control animals received the CCT vehicle without the AGN 194310 active ingredient (AGN 194310 Vehicle). Although many retinoids and retinoid analogs are light labile, this compound is relatively stable to normal light.

While this invention has been described with respect to various specific examples and embodiments, it is to be understood that the invention is not limited thereto and that it can be variously practiced with the scope of the following claims.

## Claims

1. Use of an RAR antagonist or an RAR inverse agonist for the manufacture of a medicament for the treatment of hyperlipidemia in a mammal, wherein said RAR antagonist or RAR inverse agonist has the chemical structure: 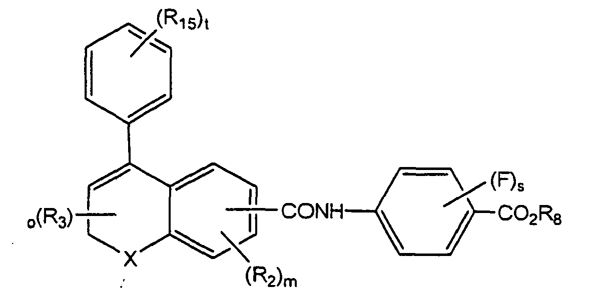 wherein X is C(R₁)₂ or O, and
R₁ is H or alkyl of 1 to 6 carbons, and;
R₂ is independently lower alkyl of 1 to 6 carbons, F, CI, Br, I, CF₃, fluoro substituted alkyl of 1 to 6 carbons, OH, SH, alkoxy of 1 to 6 carbons, or alkylthio of 1 to 6 carbons, and;
m is an integer having the value of 0-3, and;
R₃ is independently lower alkyl of 1 to 6 carbons or F, and;
o is an integer having the value of 0-3, and;
s is an integer having the value of 1-3, and;
R₈ is an alkyl group of 1 to 10 carbons or trimethylsilylalkyl where the alkyl group has 1 to 10 carbons, or a cycloalkyl group of 5 to 10 carbons, or R₈ is phenyl or lower alkylphenyl, and;
R₁₅ is independently H, F, Cl, Br, I, NO₂, N(R₈)₂, COR₈, NR₈CON(R₈)₂, OCOR₈, OR₈, CN, an alkyl group having 1 to 10 carbons, fluoro substituted alkyl group having 1 to 10 carbons, an alkenyl group having 1 to 10 carbons and 1 to 3 double bonds, an alkynyl group having 1 to 10 carbons and 1 to 3 triple bonds, or a trialkylsilyl or trialkylsilyloxy group where the alkyl groups independently have 1 to 6 carbons, and; t is an integer having the values of 0, 1, 2, 3, 4, or 5, and;
the CONH group is in the 6 or 7 position of the benzopyran, and in the 2 or 3 position of the dihydronaphthaline ring, or a pharmaceutically acceptable salt of said compound.

2. Use according to Claim 1, wherein said RAR antagonist or RAR inverse agonist has the chemical structure:

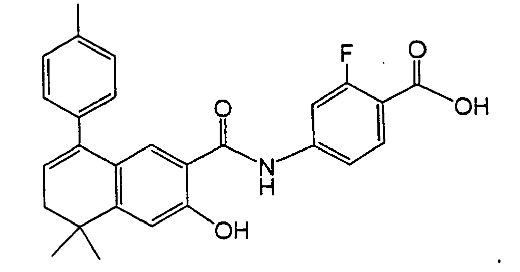

3. Use according to Claim 1, wherein said RAR antagonist or RAR inverse agonist has the chemical structure: 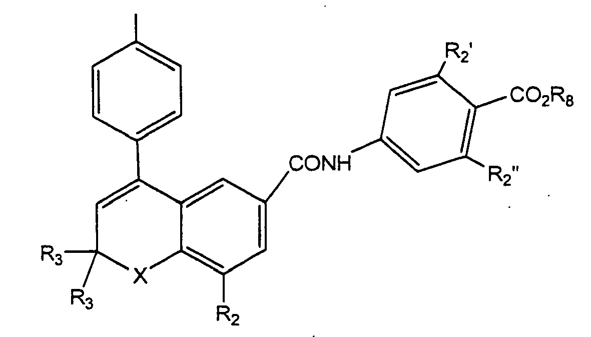 wherein X is C(CH₃)₂ or O;
R₂ is H or Br;
R₂' and R₂" independently are H or F;
R₃ is H or CH₃; and
R₈ is H, lower alkyl of 1 to 6 carbons, or a pharmaceutically acceptable salt of said compound.

4. Use of an RAR antagonist or an RAR inverse agonist for the manufacture of a medicament for the treatment of hyperlipidemia in a mammal, wherein said RAR antagonist or RAR inverse agonist has the chemical structure: 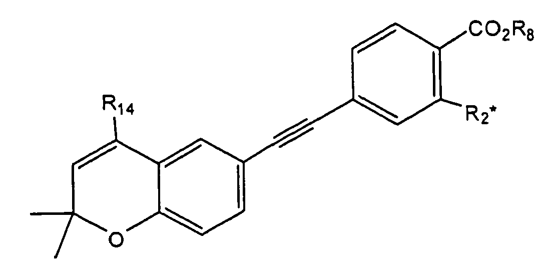 wherein R₂* is H or F;
R₈ is H, or lower alkyl of 1 to 6 carbons; and
R₁₄ is selected from the group consisting of phenyl, and 4- (lower-alkyl) phenyl, where lower alkyl has 1 to 6 carbons, or a pharmaceutically acceptable salt of said compound.

5. Use according to any one of Claims 1, 2, 3 or 4, wherein said RAR is selected from the group consisting of RARα, RARβ, and RARγ.

6. Use according to any one of Claims 1, 2, 3 or 4, wherein said RAR antagonist or an RAR inverse agonist is effective to lower the level of circulating lipid in a mammal, including a human.

7. Use according to any one of Claims 1, 2, 3 or 4, wherein said RAR antagonist or an RAR inverse agonist is effective to lower the level of circulating triglyceride in a mammal, including a human.

8. Use according to any one of Claims 1, 2, 3 or 4, wherein the step of administering said RAR antagonist or an RAR inverse agonist further prevents myocardial infarction.

9. Use according to any one of Claims 1, 2, 3 or 4, wherein the RAR antagonist or an RAR inverse agonist is administered orally.

10. Use according to any one of Claims 1, 2, 3 or 4, wherein the RAR antagonist or an RAR inverse agonist is administered topically.

11. Use according to any one of Claims 1, 2, 3 or 4, wherein the RAR antagonist or an RAR inverse agonist is administered systematically.

12. An RAR antagonist or an RAR inverse agonist for use in a method for the treatment of hyperlipidemia in a mammal, wherein said RAR antagonist or RAR inverse agonist has the chemical structure: 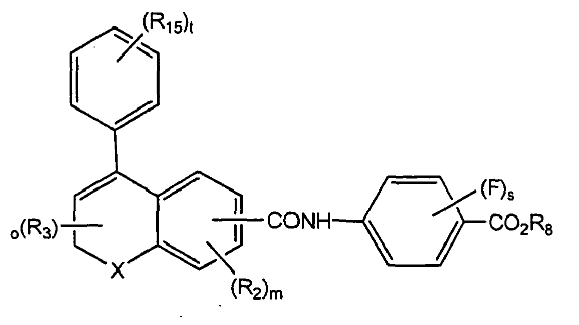 wherein X is C(R₁)₂ or O, and
R₁ is H or alkyl of 1 to 6 carbons, and;
R₂ is independently lower alkyl of 1 to 6 carbons, F, CI, Br, I, CF₃, fluoro substituted alkyl of 1 to 6 carbons, OH, SH, alkoxy of 1 to 6 carbons, or alkylthio of 1 to 6 carbons, and;
m is an integer having the value of 0-3, and;
R₃ is independently lower alkyl of 1 to 6 carbons or F, and;
o is an integer having the value of 0-3, and;
s is an integer having the value of 1-3, and;
R₈ is an alkyl group of 1 to 10 carbons or trimethylsilylalkyl where the alkyl
group has 1 to 10 carbons, or a cycloalkyl group of 5 to 10 carbons, or R₈ is phenyl or lower alkylphenyl, and;
R₁₅ is independently H, F, Cl, Br, I, NO₂, N(R₈)₂, COR₈, NR₈CON(R₈)₂, OCOR₈, OR₈, CN, an alkyl group having 1 to 10 carbons, fluoro substituted alkyl group having 1 to 10 carbons, an alkenyl group having 1 to 10 carbons and 1 to 3 double bonds, an alkynyl group having 1 to 10 carbons and 1 to 3 triple bonds, or a trialkylsilyl or trialkylsilyloxy group where the alkyl groups independently have 1 to 6 carbons, and; t is an integer having the values of 0, 1, 2, 3, 4, or 5, and;
the CONH group is in the 6 or 7 position of the benzopyran, and in the 2 or 3 position of the dihydronaphthaline ring, or a pharmaceutically acceptable salt of said compound.

13. The RAR antagonist or RAR inverse agonist according to Claim 12, wherein said RAR antagonist or RAR inverse agonist has the chemical structure:

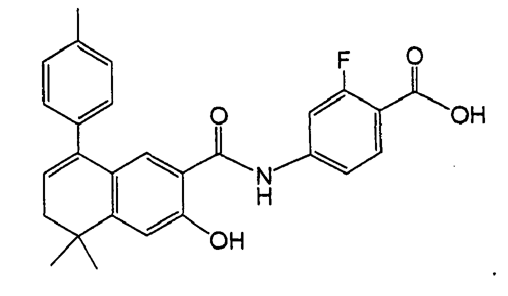

14. The RAR antagonist or RAR inverse agonist according to Claim 12, wherein said RAR antagonist or RAR inverse agonist has the chemical structure: 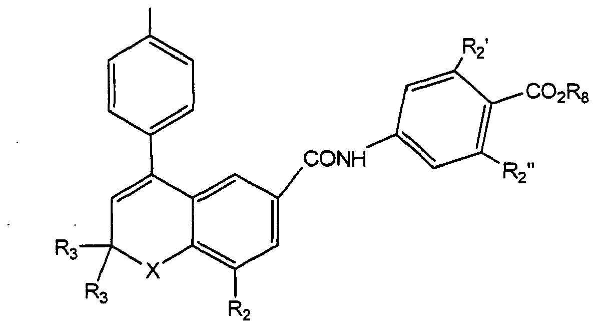 wherein X is C(CH₃)₂ or 0;
R₂ is H or Br;
R_{2'} and R_{2"} independently are H or F;
R₃ is H or CH₃; and
R₈ is H, lower alkyl of 1 to 6 carbons, or a pharmaceutically acceptable salt of said compound.

15. An RAR antagonist or an RAR inverse agonist for use in a method for the treatment of hyperlipidemia in a mammal, wherein said RAR antagonist or RAR inverse agonist has the chemical structure: 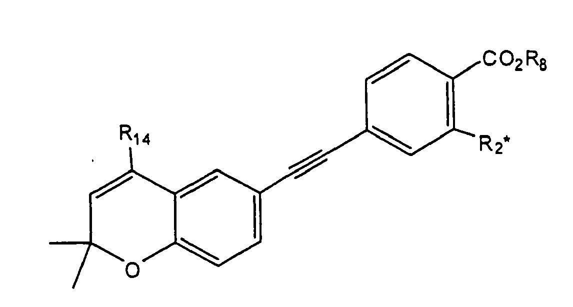 wherein R₂* is H or F;
R₈ is H, or lower alkyl of 1 to 6 carbons; and
R₁₄ is selected from the group consisting of phenyl, and 4- (lower-alkyl) phenyl, where lower alkyl has 1 to 6 carbons, or a pharmaceutically acceptable salt of said compound.

16. The RAR antagonist or RAR inverse agonist according to any one of Claims 12, 13, 14 or 15, wherein said RAR is selected from the group consisting of RARα, RARβ, and RARγ.

17. The RAR antagonist or RAR inverse agonist according to any one of Claims 12, 13, 14 or 15, wherein said RAR antagonist or an RAR inverse agonist is effective to lower the level of circulating lipid in a mammal, including a human.

18. The RAR antagonist or RAR inverse agonist according to any one of Claims 12, 13, 14 or 15, wherein said RAR antagonist or an RAR inverse agonist is effective to lower the level of circulating triglyceride in a mammal, including a human.

19. The RAR antagonist or RAR inverse agonist according to any one of Claims 12, 13, 14 or 15, wherein the step of administering said RAR antagonist or an RAR inverse agonist further prevents myocardial infarction.

20. The RAR antagonist or RAR inverse agonist according to any one of Claims 12, 13, 14 or 15, wherein the RAR antagonist or an RAR inverse agonist is administered orally.

21. The RAR antagonist or RAR inverse agonist according to any one of Claims 12, 13, 14 or 15, wherein the RAR antagonist or an RAR inverse agonist is administered topically.

22. The RAR antagonist or RAR inverse agonist according to any one of Claims 12, 13, 14 or 15, wherein the RAR antagonist or an RAR inverse agonist is administered systematically.
